# EUROPÄISCHE PATENTSCHRIFT

(11) **EP 0 787 685 B1**
(45) Veröffentlichungstag und Bekanntmachung des Hinweises auf die Patenterteilung: **29.06.2005**
(21) Anmeldenummer: 97101619.1
(22) Anmeldetag: 03.02.1997
(51) Int. Cl.: C01B 33/12, A61K 6/02, C09C 1/30

(54) **Kristalline Kieselsäuren**
Crystalline silicic acids
Acides siliciques cristallins

(30) Priorität: 05.02.1996 DE 19603838
(43) Veröffentlichungstag der Anmeldung: 06.08.1997
(73) Patentinhaber: Clariant GmbH, 65929 Frankfurt am Main (DE)
(72) Erfinder: Bauer, Harald, Dr., 50170 Kerpen (DE)

(56) Entgegenhaltungen:
- EP-A- 0 150 442
- EP-A- 0 151 295
- EP-A- 0 717 976
- ZEITSCHRIFT FUER NATURFORSCHUNG , Bd. 28b, 1973, TUEBINGEN, DE, Seiten 234-238, XP002029725 G. LAGALY ET AL.: "Ueber eine neue kristalline Kieselsäure der Zusammensetzung H2Si14O29.5H2O mit Schichtstruktur und Befähigung zur Bildung von Intercalationsverbindungen"
- GIT FACHZEITSCHRIFT FUER DAS LABORATORIUM, Bd. 28, Juni 1984, DARMSTADT, DE, XP000646328 K. BENEKE ET AL.: "Kristalline Kieselsäuren"

## Beschreibung

Die vorliegende Erfindung betrifft kristalline Kieselsäuren und ihre Verwendung sowie die damit hergestellten Produkte.

Kristalline Kieselsäuren sind seit längerem bekannt. Sie können durch Umsetzung der entsprechenden Natriumsilikate mit Säuren erhalten werden. Die DE-A-34 00 130 beschreibt die Herstellung eines kristallinen Natriumsilikates mit charakteristischem Röntgenbeugungsdiagramm (SKS-1) sowie die Herstellung der entsprechenden kristallinen Kieselsäure aus diesem Natriumsilikat, die als SKS-1H bezeichnet wird. Hergestellt wird nach der vorgenannten Schrift die kristalline Kieselsäure SKS-1H durch Umsetzung von SKS-1 mit Mineralsäuren, bevorzugt mit einem Säureüberschuß. Auch die wiederholte Umsetzung von SKS-1 mit Mineralsäuren ist danach möglich. Weiterhin kann gemäß der DE-A-34 00 130 die Umsetzung von SKS-1 mit Salzsäure zu SKS-1H in Form einer Titration erfolgen.

Die DE-A-34 00 132 beschreibt analog zur DE-A-34 00 130 die Herstellung von SKS-1 und eines weiteren Natriumsilikates, welches als SKS-2 bezeichnet wird. Für beide Silikate sind die charakteristischen Röntgenbeugungsdaten angegeben. Es wird beschrieben, daß durch Extraktion bzw. durch Umsetzung von SKS-1 mit Salzsäure SKS-1H und entsprechend aus SKS-2 die Kieselsäure SKS-2H hergestellt wird. Beide Kieselsäuren lassen sich durch ihre Röntgenbeugungsdiagramme charakterisieren.

Die DE-A-35 23 364 beschreibt die Herstellung von SKS-1 und SKS-2 aus Natronwasserglas auf hydrothermalem Weg.

Beneke und Lagaly (GIT (1984) 516-527) beschreiben die Herstellung von SKS-1, SKS-2, SKS-1H und SKS-2H und einige Einsatzmöglichkeiten. SKS-2H wird auch von Lagaly (Z. Naturforsch 28b (1973) 234-238) beschrieben.

Eugster (Science 157 (1967) 1177-1180) beschreibt eine Kieselsäure SH, die durch Ansäuern der Minerale Magadiit und Kenyait erhalten wird.

Für eine Reihe von Anwendungsbereichen ist es sinnvoll, Produkte zur Verfügung zu haben, die unter bestimmtem Bedingungen Transparenz zeigen.

Es ist daher Aufgabe der vorliegenden Erfindung, kristalline Kieselsäuren zur Verfügung zu stellen, die transparent sind.

Diese Aufgabe wird gelöst durch eine transparente kristalline Kieselsäure SKS-1H und/oder SKS-2H, die dadurch gekennzeichnet ist, daß sie nach Siebanalyse weniger als 20 Gew.-% Teilchen mit einer Korngröße von weniger als 53 µm aufweist.

Die vorliegende Aufgabe wird ebenfalls gelöst durch die Verwendung von transparenten kristallinen Kieselsäuren in der Zahnmedizin.

Bevorzugt werden die transparenten kristallinen Kieselsäuren zur Herstellung von Füllstoffen in der Zahnmedizin verwendet.

Für diese Verwendung weist die transparente kristalline Kieselsäure nach Siebanalyse weniger als 20 Gew.-% Teilchen mit einer Korngröße von weniger als 53 µm auf.

Bevorzugt handelt es sich für diese Verwendung um die kristalline Kieselsäure SKS-1H und/oder SKS-2H.

Die kristallinen Kieselsäuren werden insbesondere in Zahnersatz, Zahnfüllungen, Zahnverblendungen und/oder Dentalzementen verwendet.

Die Erfindung betrifft ebenfalls einen Füllstoff für die Zahnmedizin, enthaltend eine kristalline Kieselsäure.

Bevorzugt ist dieser Füllstoff transparent.

Bevorzugt weist die im Füllstoff enthaltene kristalline Kieselsäure nach Siebanalyse weniger als 20 Gew.-% Teilchen mit einer Korngröße von weniger als 20 % auf.

Bevorzugt handelt'es sich bei der kristallinen Kieselsäure für den Füllstoff um SKS-1H und/oder SKS-2H.

In Kunststoffen werden Füllstoffe zur Beeinflussung der Eigenschaften eingesetzt. Füllstoffe können dazu beitragen, daß die Festigkeit oder Abriebfestigkeit gesteigert wird. Füllstoffe dienen auch der Veränderung der optischen Eigenschaften. Pigmente lassen das Einfärben von Kunststoffen zu oder ergeben mehr oder minder transparente und transluzente Produkte.

Insbesondere in der Zahnmedizin werden Kunststoffmassen eingesetzt, die in dem Erscheinungsbild dem natürlichen Zahn gleichen sollen. Dazu werden spezielle Füllstoffe eingesetzt.

Füllstoffe werden weiterhin eingesetzt, um eine Materialeinsparnis hinsichtlich Polymer zu erzielen.

Die erfindungsgemäß eingesetzten Schichtsilikate lassen sich bevorzugt aus den Natriumschichtsilikaten SKS-1 und SKS-2 herstellen. Deren Herstellung auf hydrothermalem Wege ist in den vorgenannten Schriften DE-A-34 00 130, DE-A-34 00 132 und DE-A-35 23 364 beschrieben. Natriumschichtsilikate anderer Herkunft sind dagegen nicht geeignet. Wird beispielsweise das Schichtsilikat SKS-6 (Fa. Hoechst Aktiengesellschaft, Frankfurt/M.) mit Säure in die kristalline Kieselsäure SKS-6H umgewandelt, so ergibt dies ein Produkt mit einer wesentlich geringeren, d.h. praktisch keiner Transparenz.

Die Umsetzung des Natriumsilikates kann grundsätzlich mit jeder Säure erfolgen. Bevorzugt werden jedoch Mineralsäuren und insbesondere Schwefelsäure. Während bei einer normalen Brönstedt-Säure-Base-Reaktion lediglich eine Protonierung der Base erfolgt, muß bei Schichtsilikaten auch der Ionenaustauscheffekt berücksichtigt werden.

Eine einmalige Umsetzung mit stöchiometrischer Menge Säure reicht daher nicht, um den Natriumgehalt genügend herabzusetzen. Vielmehr ist es günstig, eine erste Umsetzung mit geringem Säureüberschuß durchzuführen, den Feststoff dann abzufiltrieren und gut zu waschen. Mit dem Waschprozeß senkt man zunächst die Konzentration an Natrium-Ionen. Letztere stehen in einem Ionenaustauschgleichgewicht mit dem Schichsilikatgerüst. Das gut ausgewaschene SKS-1H mit noch zu hohem Natriumgehalt wird dann noch einmal oder gegebenenfalls mehrfach überstöchiometrisch mit Säure versetzt, filtriert und gewaschen.

Während die Filtration und das Waschen im Labormaßstab mit einer Filternutsche durchgeführt werden können, muß bei größeren Mengen eine Filterpresse eingesetzt werden. In einem ersten Schritt wird der Filterkuchen mit entionisiertem Wasser im Verhältnis Festkörper : Wasser von 1 : 12,5 bis 1 : 50 gewaschen. Da der Festkörper durch den Ionenaustausch aufquillt und somit das Waschen verhindert, wird der Filterkuchen nochmals in Wasser aufgeschlämmt und der Filtrations- und Waschprozeß entsprechend genügend oft wiederholt. Alternativ kann auch eine Zentrifuge verwendet werden.

Das feuchte Produkt wird getrocknet. Dies kann im Trockenschrank bei Temperaturen bis 150 °C, bevorzugt bei 80 °C erfolgen. Es ist jedoch auch die Trocknung im Heißluftstrom möglich, z.B. einem Spin-flash-Trockner der Fa. Anhydro oder einem Labortrockner der Fa. Retsch, der nach dem Wirbelbett-Prinzip arbeitet.

Die getrocknete Kieselsäure muß noch gebrochen werden. Grundsätzlich können Kieselsäuren mit verschiedenen Kornspektren eingesetzt werden. Es wird jedoch ein Kornspektrum bevorzugt, das die Einarbeitung in die organische Phase erleichtert und mit dem die benötigte Transparenz in organischer Umgebung erreicht wird. Die Kieselsäure soll die organische Komponente durch Kapillarkräfte oder Adsorption aufnehmen und binden können und eine optimale Konsistenz für Extrusion, Walzen u. dgl. ergeben.

Erfindungsgemäß beträgt daher in der kristallinen Kieselsäure der Feststoffanteil mit einem Teilchendurchmesser von weniger als 53 µm weniger als 20 Gew.-% (nach Siebanalyse). Je kleiner der relative Anteil an Teilchen mit einem Teilchendurchmesser von weniger als 53 µm ist, desto höher ist die Transparenz der Kieselsäure.

Die Transparenz der Proben wird in einem organischen Lösungsmittel bestimmt. Die Substanz wird in Toluol suspendiert und dann in einem Photometer vermessen. Die gemessenen Transparenzwerte lassen sich gut auf Polymere, z.B. Polyacrylate oder Polyacryl-Copolymere übertragen. Derartige Polymere werden im Dentalbereich für Zahnzemente oder Dentalprothesen verwendet.

### Beispiel 1

Es werden 50 kg SKS-1 (Glühverlust: 11,8 %) in 230 kg entionisiertem Wasser mit einer Temperatur von 20 °C suspendiert. Dann werden 3,604 kg Schwefelsäure (96 Gew.-%) zudosiert. Der pH-Wert der Lösung beträgt 2,8. Es wird 2 Stunden nachgerührt.

Die Suspension wird geteilt und in eine Filterpresse gepumpt, abfiltriert und der Filterkuchen mit entionisiertem Wasser gewaschen (Volumen jeweils ca. 500 l). Die beiden Feststofffraktionen werden vereinigt und mit 230 kg Wasser versetzt. Wiederum wird die Suspension geteilt und in einer Filterpresse filtriert. Dieser Zyklus wird noch einmal wiederholt.

Um den Natriumgehalt noch weiter abzusenken, wird der gewaschene Feststoff abermals mit Wasser versetzt und mit 0,130 kg Schwefelsäure (96 Gew.-%) versetzt, nachgerührt, filtriert und mit 500 l entmineralisiertem Wasser gewaschen.

Der Filterkuchen wird im Trockenschrank bei 80 °C 15 Stunden getrocknet. Es werden 25 kg SKS-1H (Glühverlust: ca 3 %) erhalten. Das Material wird in einer Hammermühle (Fa. Isma, Siebweite 1,5 mm) gemahlen (Siebanalyse: > 750 µm: 6,6 Gew.-%, > 425 µm: 26,2 Gew.-%, > 212 µm: 49,2 Gew.%, > 150 µm: 58,1 Gew.-%, > 53 µm: 80,5 Gew.-%, < 53 µm: 19,5 Gew.-%). Es wurde der Weißgrad, der Trockenverlust (ca. 1,2 %), die BET-Oberfläche (bei Raumtemperatur: 50 m²/g, nach Ausheizen bei 150 °C: 59 m²/g), der Natriumgehalt (530 ppm) und der Sulfatgehalt (190 ppm) bestimmt. Die Transparenz einer 20 %-igen toluolischen Suspension betrug 74,8 %. Ein Röntgenpulverdiffraktogramm ergibt folgende Reflexe:

| Netzebenenabstand [Angström] | rel. Intensität [%] |
|---|---|
| 17,704 | 16,72 |
| 8,889 | 8,26 |
| 7,303 | 6,00 |
| 3,673 | 8,90 |
| 3,403 | 100,00 |
| 3,373 | 81,88 |
| 1,839 | 20,93 |

### Beispiel 2

Es werden 210 g Schwefelsäure (96 Gew.-%) in 5 l Wasser gegeben. Nach dem Erkalten werden 3,092 kg SKS-1 (Glühverlust: 10,3 %) zugegeben und 2 Stunden bei einem pH-Wert von 4,5 gerührt. Auf einer Labornutsche wird abgesaugt und mit 15 l demineralisiertem Wasser gewaschen. Die kristalline Kieselsäure wird im Trockenschrank 15 Stunden bei 80 °C getrocknet und enthält weniger als 30 ppm Sulfat. Die Reflexe aus dem Röntgenpulverdiffraktogramm entsprechen denen von Beispiel 1. Die Transparenz des Produktes betrug 71,9 %.

### Bestimmung der Transparenz

In eine Glasflasche werden 16 g Toluol und 4 g Probesubstanz (20 %ige Suspension) eingewogen und eine Stunde bei Raumtemperatur gerührt. Die Suspension wird mit einer Pipette in eine Quarzküvette (d = 1mm) eingefüllt. Die Transparenzmessung erfolgt in einem Photometer (Typ: ELKO 2, Fa. Carl. Zeiss, Filter Nr. S51E67). Als Referenzlösung dient demineralisiertes Wasser, der Messwert für die Transparenz wird direkt am Gerät abgelesen.

Die in den Vergleichsbeispielen genannten Produkte werden nach dem Stand der Technik (DE-A-34 00 130 und DE-A-34 00 132) bzw. durch Ansäuern von handelsüblichem SKS-6 erhalten.

**Tabelle**

| Transparenzwerte verschiedener Silikate in Toluol | |
|---|---|
| Silikat-Typ | Transparenzwert |
| SKS-1H | 74,8 % (erfindungsgemäß) |
| SKS-2H | 71,6 % (erfindungsgemäß) |
| SKS-6H | 4,85 % (Vergleichsbeispiel) |
| SKS-1 | 59,6 % (Vergleichsbeispiel) |
| SKS-2 | 31,3 % (Vergleichsbeispiel) |

### Anwendung

Zur Herstellung einer Zahnmasse wird eine kristalline Kieselsäure SKS-1H mit weiteren Füllstoffen und benötigten Substanzen innig vermischt, etwa durch Einwalzen. Das SKS-1H bindet dabei das Monomer und erleichtert das Mischen. Die so erhaltene künstliche Zahnmasse besitzt aufgrund des Gehaltes an SKS-1H eine dem natürlichen Zahn ähnliche Transparenz.

## Patentansprüche

1. Transparente kristalline Kieselsäure SKS-1H und/oder SKS-2H, **dadurch gekennzeichnet, dass** sie nach Siebanalyse weniger als 20 Gew.-% Teilchen mit einer Korngröße von weniger als 53 µm aufweist.

2. Verwendung der transparenten kristallinen Kieselsäure nach Anspruch 1 in Zahnersatz, Zahnfüllungen, Zahnverblendungen, Dentalzementen oder als Füllstoffe für die Zahnmedizin.

## Claims

1. A transparent crystalline silica SKS-1H and/or SKS-2H, which exhibits, after sieve analysis, less than 20% by weight of particles with a particle size of less than 53 µm.

2. The use of the transparent crystalline silica as claimed in claim 1 in dentures, tooth fillings, dental veneers or dental cements or as fillers for dentistry.

## Revendications

1. Acide silicique cristallin transparent SKS-1H et/ou SKS-2H, **caractérisé en ce qu'**il présente, d'après l'analyse granulométrique, moins de 20 % en poids de particules ayant une granulométrie inférieure à 53 µm.

2. Utilisation de l'acide silicique cristallin transparent selon la revendication 1 dans la prothèse dentaire, les obturations dentaires, les couronnes dentaires, les ciments dentaires ou comme matières de remplissage pour la médecine dentaire.
